# EUROPEAN PATENT APPLICATION

(11) **EP 2 666 870 A1**
(43) Date of publication of application: **27.11.2013**
(21) Application number: 12305564.2
(22) Date of filing: 23.05.2012
(51) Int. Cl.: C12Q 1/68

(54) **Method for differential treatment of nucleic acid contents of a sample, sample enrichment, kit and uses thereof**

(71) Applicant: Pathoquest, 75724 Paris Cedex 15 (FR); Ecole Nationale Veterinaire D'Alfort, 94704 Maisons-Alfort (FR)
(72) Inventor: Eloit, Marc, 75006 Paris (FR)
(74) Representative: Desaix, Anne

(57) **Abstract**

The invention relates to a method and a kit for *in vitro* differential treatment of nucleic acid molecules contained in a biological sample and comprising a first subpopulation of free nucleic acids of higher eukaryotic cells and at least one further subpopulation of nucleic acids of microorganism(s) wherein said microorganism(s) nucleic acids are packaged nucleic acids that represents quantitatively a minor group of the nucleic acids of the sample, comprising at least the steps of:
a. exposing the biological sample to conditions enabling the modification of the 3'- and/or 5'- extremities of degraded nucleic acid molecules of the first nucleic acids subpopulation in order to impair their ligation capacity;
b. extracting nucleic acid molecules of the sample and thereby recovering degraded nucleic acid molecules of the first nucleic acids subpopulation having modified 3'- and/or 5'- extremities and nucleic acid molecules of the further subpopulation having non-modified 3'- and/or 5'- extremities.

## Description

The invention relates to the field of sample preparation for advanced nucleic acid investigation methods, such as PCR-based methods, microarrays or high-throughput sequencing.

The invention is more particularly directed to a method for the differential treatment of at least two nucleic acid subpopulations that can be found within a sample, at least one of these subpopulations being lower in quantity than the others or lower in quantity than a quantitatively significantly dominant subpopulation, and referred to as the "quantitatively minor" subpopulation of nucleic acids. As a result of the sample preparation method of the invention, the invention also concerns a method of enrichment of at least one minor nucleic acid subpopulation of the sample that can be achieved through amplification of the nucleic acid contents of the prepared sample, in particular through, but not restricted to, Multiple Displacement Amplification (MDA). The sample preparation method of the invention therefore allows for differential or selective amplification of at least one nucleic acid subpopulation within a sample.

The invention also relates to a kit for performing such a method, and to the use of the kit and methods of the invention for analyzing the nucleic acid contents of the assayed samples. The invention is of particular interest in the area of *in vitro* microorganisms detection, in particular when applied to pathogens detection in biological samples.

New tools for microorganisms (especially bacteria, viruses, fungi) detection directly from biological samples are emerging: they include microarrays and High-Throughput Sequencing (HTS). When used to detect the occurrence of specific microorganisms within a sample, in particular pathogenic ones, problems arise in the sense that these techniques need amounts of pathogen nucleic acids (NAs) that cannot be easily derived from direct purification from the samples.

One approach that has been developed to identify the presence of pathogen nucleic acids within a sample is to perform sequence-based specific

PCR tests aimed at detecting predefined targets. However, this approach requires the definition of particular, already known targets prior to their detection.

A preferred approach would allow performing a universal test suitable for nucleic acid detection on any pathogenic targets, including unknown targets. Such an approach would benefit of an enrichment of the sample in pathogen sequences followed by sequence-based identification of the targets. To date, different techniques have been developed for enrichment, that use random amplification of nucleic acids like random Polymerase Chain Reaction (random PCR), Rolling Circle Amplification (RCA) and Multiple Displacement Amplification (MDA), with the view to sufficiently amplify nucleic acids available in a sample to envision their subsequent detection.

These techniques are especially useful in the detection of contamination of samples, especially biological samples, by microbial organisms, especially pathogen microorganisms.

However, sometimes the scarcity of target nucleic acids present in the samples to be assayed, in combination with a relatively high level of hosts genomic DNA, remains a main obstacle to the identification of microorganisms in biological samples. As it can be seen from Table 1, contaminating cellular DNA is an issue in pathogens detection experiments, due to its overwhelming abundance (up to 95% of sequence information).

**Table 1: Contaminating cellular DNA hampers pathogen detection in high-throughput sequencing experiments. About hundred million sequence reads were obtained from each biological sample (human plasma), a vast majority of which represents contaminating host cellular DNA of human origin.**

| **Samples** | **Depth of sequencing (Number of sequence reads)** | **Percentage of sequence reads of human origin** |
|---|---|---|
| **110250** | 96,436,140 | 92.64% |
| **110253** | 80,416,672 | 89.64% |
| **110254** | 81,859,146 | 95.69% |
| **110255** | 98,174,114 | 77.12% |

In the initial developments of the above-mentioned techniques, all nucleic acids from the samples were extracted and random-amplified. The drawback of this methodology is the simultaneous amplification of both microorganism and host nucleic acids. This limits the sensitivity of downstream microorganisms nucleic acids detection because of the background amplified host nucleic acids levels: for example detection with microarrays is less sensitive, and HTS needs deeper sequencing to reach adequate levels of sensitivity. The significant supplemental time and resources required for achieving deeper sequencing are economically burdensome enough for preventing this technique to be used as a routine tool for detecting microorganisms, in particular pathogens.

Concerning the issue of simultaneous amplification of both microorganism and host nucleic acids, even the use of the very powerful MDA technique (Silander K, Saarela J.(2008). Methods Mol Biol.;439:1-18.; Hughes S, Arneson N, Done S, Squire J. (2005). Prog Biophys Mol Biol.;88(1):173-89.; Lasken RS, Egholm M. (2003). Trends Biotechnol.;21(12):531-5.) has not come to an acceptable solution as to the possibility of increasing the signal-to-noise ratio of such experiments.

Among the different techniques allowing random nucleic acids amplification, MDA is a technique that has become a method of choice for many biomedical applications that require high amounts of high quality DNA derived from minute amounts of source nucleic acids. MDA has generally been used for the purpose of amplification of whole genomic DNA (Whole Genome Amplification or WGA).

To bypass the above-mentioned drawback of simultaneous amplification of both microorganism and host nucleic acids, degradation by hydrolysis of host DNA by nucleases has been proposed (Erlandsson et al., The Microbial Detection Array Combined with Random Phi29-Amplification Used as a Diagnostic Tool for Virus Detection in Clinical Samples, PLoS one, August 2011, Vol 6, Issue 8), mainly for the detection of viral nucleic acids, that are protected by capsids.

A pre-requisite for efficient amplification by the MDA technique is the annealing of multiple nucleotidic primers on a single DNA molecule. With a proper DNA polymerase the previous situation allows strand displacement when the nascent 5'-juxtaposed elongation chain reaches the following 3'- primed-chain, this explains why MDA is only efficient on long linear (not less than 2kb) and circular molecules.

The use of MDA with high-fidelity DNA polymerase with high displacement strand activity such as the DNA polymerase of phage phi29 in the detection of small genomes like viral genomes requires prior ligation of nucleic acids, so as to provide concatemers of high molecular weights that are more efficiently amplified by high-fidelity DNA polymerase with high displacement strand activity. A state of the art protocol includes: 1) optional treatment of the sample with nucleases to digest host free nucleic acids, 2) extraction of nucleic acids from the sample, 3) reverse transcription step (to take into account that RNA is the genetic material of some microorganisms), 4) ligation of nucleic acids, 5) amplification by MDA, 6) detection of microorganisms nucleic acids.

However, as shown by the inventors, this protocol does not solve the drawback of simultaneous amplification of both microorganism and host nucleic acids, and the "low signal over noise" issue remains.

Within the frame of the present invention, the inventors surprisingly found, as shown in Table 2, that hydrolysis of host nucleic acids does not prevent its successful amplification by MDA following ligation. As shown in Table 2, such digested nucleic acids remain amplified, albeit less efficiently than the non-digested DNA.

**Table 2: Nucleases digestion of contaminating cellular DNA does not prevent its amplification by MDA. Two biological samples (human plasma) were submitted or not to nucleases digestion prior extraction, ligation and amplification by the MDA technique. The remaining cellular contamination was evaluated by quantitative real-time PCR using the human β-globin gene as a marker. Duplicate values for the Cycle Threshold parameter (C_{T}; PCR cycle at which the β-globin sequence is detected) are indicated. Although a significant reduction in contaminating cellular DNA is observed (6.2 and 24 fold-reductions in samples #2 and #1, respectively) following nucleases treatment, the nucleases-treated contaminating DNA remains amplified by the MDA procedure.**

| **Samples** | **Untreated (C_{T})** | **Nucleases-Treated (C_{T})** | **Fold difference 2^{(Nuc. Treat.C}_{T} ^{-Untreat.C}_{T})** |
|---|---|---|---|
| Sample #1 | 31.54 / 31.54 | 36.23 / 36.03 | 24 |
| Sample #2 | 24.97 / 25.00 | 27.67 / 27.59 | 6.2 |

The Cycle Threshold parameter (C_{T}) is a parameter used in real-time PCR, which defines at which PCR cycle a specific amplification of the target is detected.

The inventors' experiments allowed concluding that host nucleic acid did not disappear as a target for amplification using known state of the art protocols. A possible explanation is that full digestion of NA is a difficult task to achieve, as circulating host DNA is protected by histones and non-histones proteins involved in chromatin structure. In addition, the above-proposed protocol possesses another drawback, which is that high concentrations of nucleases are potentially detrimental to microorganism nucleic acids, whose packaging inside bacteria, fungi or viruses can be imperfect.

It is also another object of the invention to design a method allowing enrichment of pathogens nucleic acids within a sample with a reduced amount of steps, rendering such a method quick and easy to perform, and minimizing the amount of necessary manipulations of the sample.

The inventors have therefore designed a method which overcomes at least some of the prior art's drawbacks, based upon an adequate sample preparation. The invention therefore relates to a method for the *in vitro* differential treatment of nucleic acid molecules contained in a biological sample and comprising a first subpopulation of free nucleic acids of higher eukaryotic cells, such as genomic nucleic acids, in particular genomic DNA, that represents the major group of the nucleic acids of the sample, and at least one further subpopulation of nucleic acids of microorganism(s) wherein said microorganism(s) nucleic acids are packaged nucleic acids that represent(s) quantitatively a minor group of the nucleic acids of the sample, comprising the steps of:
a. optionally, exposing the biological sample to degradation conditions to enable degradation of free nucleic acid molecules and production of degraded nucleic acid molecules thereof;
b. exposing the biological sample to conditions enabling the modification of the 3'- and/or 5'- extremities of degraded nucleic acid molecules of the first nucleic acids subpopulation in order to impair their ligation capacity;
c. extracting nucleic acid molecules of the sample and thereby recovering degraded nucleic acid molecules of the first nucleic acids subpopulation having modified 3'- and/or 5'- extremities and nucleic acid molecules of the further subpopulation having non-modified 3'- and/or 5'- extremities;
d. optionally, converting nucleic acid molecules from step c. that are not DNA molecules, into cDNA or DNA molecules,
e. performing a ligation step on nucleic acid molecules recovered in step c. and/or step d. to obtain nucleic acid molecules longer than 2000 nucleotides, and recovering the same.

In a particular embodiment, the extraction of nucleic acid molecules of the sample set forth in step c. is preceded with a step of elimination of any of pre-treatment reagents possibly used in steps a. and b.

By "nucleic acid molecule" or "polynucleotides", it is meant any linear or circular chain of nucleotides, each nucleotide being formed by the association of a pentose sugar (ribose or deoxyribose) and a nucleobase (resulting altogether in a nucleoside), and at least one phosphate group. Nucleic acid molecules can for example be DNA (deoxyribonucleic acid) or RNA (ribonucleic acid). These molecules can comprise non-standard or modified nucleosides.

By "subpopulation of free nucleic acids", it is meant that the nucleic acid molecules of said subpopulation are: either not contained into cells and therefore not protected by cell walls or cell structures within the biological sample, or are rendered free after a step aimed at rendering the nucleic acid molecules accessible to a later degradation, as disclosed and detailed hereafter.

Nucleic acid molecules of the invention are generally double-stranded or single-stranded or sometimes also comprise a triple or quadruple strand portion, and can range in size from about dozens of nucleotides to millions of nucleotides, especially when the considered nucleic acid molecules encompass whole genomes.

The sugars and phosphates in nucleic acids are connected to each other in an alternating chain (sugar-phosphate backbone) through phosphodiester bonds. In conventional nomenclature, a phosphodiester bond is formed when the phosphate group on the 5' position of the sugar ring (ribose or deoxyribose) of a first nucleotide, attaches to the hydroxyl group on the 3' position of the sugar ring of a next nucleotide thereby constituting a dimer or dinucleotide. This gives nucleic acids directionality, and the extremities of nucleic acid molecules are referred to as 5'- extremity and 3'- extremity. *In vivo,* DNA and RNA are synthesized in the 5' to 3' direction. DNA chain synthesis involves a condensation reaction between the 5' phosphate group of the current nucleotide with the 3' hydroxyl group of the previous nucleotide.

By "biological sample" it is meant a sample originating from the sampling of biological tissue(s) or fluid(s), especially body tissue(s) or fluid(s), which is therefore substantially constituted of cells, for example bodily fluid such as a cerebrospinal fluid, saliva, mucus, urine, serum, plasma or blood sample, or include a cell lysate of the same origin, and/or include a conditioned culture medium, and is optionally derived from a tissue (*e.g*., a tissue homogenate), a biopsy, and/or a tumor.

It is alternatively meant a sample originating from the sampling of non-biological material, such as water, that is susceptible to be contaminated by both nucleic acid(s) belonging to an organism of the range of higher eukaryotes, and microorganism(s) nucleic acid(s).

Therefore, all samples obtained and prepared in the context of the present invention originate from a source suspected to contain nucleic acids of metazoans or higher eukaryotic cells, especially of animal, human or plant cells and nucleic acids of one or many different microorganisms.

According to a particular embodiment, the biological sample is obtained from a "host", meaning any organism or tissue of the eukaryotic range, of an animal or a plant, in particular of mammalian, especially human, and the above-mentioned first subpopulation then comprises mainly or contains essentially or consists of genomic nucleic acids, especially DNA, from said host.

According to the present invention, at least two nucleic acid subpopulations originating from a same sample are differentially treated. A "first subpopulation" which represents the quantitatively dominant subpopulation of nucleic acids of the sample comprises mainly or contains essentially or consists of genomic nucleic acids and at least a "further subpopulation", which represent(s) quantitatively a minor group of the nucleic acids of the sample, contains microorganism nucleic acids.

In a particular embodiment, there are many so-called "further subpopulation", *i.e.*, multiple groups of nucleic acids belonging to different microorganisms are present in the sample and each of said groups is quantitatively minor with respect to the "first subpopulation".

In another particular embodiment there are two subpopulations, *i.e.*, a "first subpopulation" and a "second subpopulation".

By "genomic nucleic acids", it is meant nucleic acids belonging to the genome of a cell or a microorganism, or associated with the transcription of said genome within a cell or microorganism. Genomic nucleic acids can therefore be, for example, genomic DNA or mRNAs.

The genome of a cell is considered to be the DNA of an organism especially of a complex eukaryotic organism, that carries all the information for all the proteins the organism will ever synthesize, and more generally the genome contains all the information necessary for the survival of said cell. Genomic DNA can be chromosomal DNA or plasmidic DNA, with the proviso that said plasmidic DNA originates from the genome of the considered cell, as defined herein. Genomic DNA molecules (gDNA) can be either mitochondrial gDNA, chloroplastic gDNA or nuclear gDNA or a combination thereof. Within the context of the invention gDNA can also comprise circular DNAs or plasmids.

The "genomic nucleic acids" can originate from the host providing the sample for preparation according to the invention. Accordingly it can be "metazoan nucleic acids" or "higher eukaryote nucleic acids" especially nucleic acids of animal especially human, or plant cells.

Alternatively the "genomic nucleic acids" can be "microorganisms nucleic acids".

By "microorganism" it is meant microscopic organisms that are multicellular, unicellular or acellular, and include viruses, prokaryotic organisms such as bacteria and archae or lower eukaryotic organisms such as fungi and protozoan parasites. In a particular embodiment it is a virus, a bacterium, a fungus or a parasite.

In a particular embodiment, "microorganism nucleic acids" means nucleic acids belonging to the genome of a microorganism, or associated with the replication or transcription of such a genome of microorganism, that are considered as exogenous with respect to the above-mentioned genomic nucleic acids of metazoans or complex organism, *i.e.*, host providing the sample to be prepared according to the invention. Microorganism nucleic acids can therefore consist, for example, of virus RNA genomes or virus DNA genomes, double- or single- stranded, either linear or circular.

According to a particular embodiment, microorganisms are pathogenic microorganisms.

Microorganisms can for example belong to any one of the following viral families, including DNA viruses families, such as: Adenoviridae, Herpesviridae, Papillomaviridae, Polyomaviridae, Poxviridae, Hepadnaviridae, Parvoviridae, or RNA viruses families, such as Astroviridae, Bunyaviridae, Caliciviridae, Picornaviridae, Coronoviridae, Flaviviridae, Togaviridae, Orthomyxoviridae, Arenaviridae, Bunyaviridae, Filoviridae, Paramyxoviridae, Rhabdoviridae, Retroviridae, in particular lentiviruses, and Anelloviridae.

Exemplary microorganisms may therefore be viruses, especially viruses of humans, encompassing DNA viruses including Adenoviruses, Herpex simplex types 1 or 2, Varicella-zoster virus, Epstein-barr virus, Human cytomegalovirus, Human herpesvirus of type 8, Human papillomaviruses, Polyomaviruses, BK virus, JC virus, Smallpox, Hepatitis B virus, human bocavirus, Parvovirus B19, and RNA viruses such as Human astrovirus, Norwalk virus, coxsackievirus, Hepatitis A virus, poliovirus, rhinovirus, Hepatitis C virus, Flaviviridae such as the Yellow Fever virus (YFV) or dengue virus or West Nile virus (WNV), Rubella virus, Hepatitis E virus, Papillomaviruses, Human immunodeficiency virus (HIV), Influenza virus, Guanarito virus, Junin virus, Lassa virus, Machupo virus, Sabia virus, Crimean-Congo hemorrhagic fever virus, Ebola virus, Marburg virus, Measles virus, Mumps virus, Parainfluenza virus, Respiratory syncytial virus, Human metapneumovirus, Rabies virus, Hepatitis D virus, Rotavirus, Astroviruses, Enteroviruses, Noroviruses, GI and GII viruses and Mononegavirales including paramyxoviridae.

Microorganisms can also be either Gram-positive and Gram-negative bacteria belonging to any one of the following genera: *Bacillus, Bordetella, Borrelia*, *Brucella*, *Campylobacter, Chlamydia, Chlamydophila, Clostridium, Corynebacterium, Enterococcus, Escherichia, Francisella, Haemophilus, Helicobacter, Legionella, Leptospira, Listeria, Mycobacterium, Mycoplasma, Neisseria, Pseudomonas, Rickettsia, Salmonella, Shigella, Staphylococcus, Streptococcus, Treponema, Vibrio, Yersinia.*

Exemplary microorganisms may therefore be one of the following species: *Escherichia species, Enterococcus species, Salmonella species, Campylobacter species, V. vulnificus, L. monocytogenes, H. pylori, V. cholera, Clostridium species, Shigella species, mycobacteria, or be in particular: Bacillus anthracis, Bordetella pertussis, Borrelia burgdorferi, Brucella abortus, Brucella canis, Brucella melitensis, Brucella suis, Campylobacter jejuni, Chlamydia pneumoniae, Chlamydia trachomatis, Chlamydophila psittaci, Clostridium botulinum, Clostridium difficile, Clostridium perfringens, Clostridium tetani, Corynebacterium diphtheriae, Enterococcus faecalis, Enterococcus faecium, Escherichia coli, Francisella tularensis, Haemophilus influenzae, Helicobacter pylori, Legionella pneumophila, Leptospira interrogans, Listeria monocytogenes, Mycobacterium leprae, Mycobacterium tuberculosis, Mycobacterium ulcerans, Mycoplasma pneumoniae, Neisseria gonorrhoeae, Neisseria meningitidis, Pseudomonas aeruginosa, Rickettsia rickettsii, Salmonella typhi, Salmonella typhimurium, Shigella sonnei, Staphylococcus aureus, Staphylococcus epidermidis, Staphylococcus saprophyticus, Streptococcus agalactiae, Streptococcus pneumoniae, Streptococcus pyogenes, Treponema pallidum, Vibrio cholerae, Yersinia pestis.*

Microorganisms can for example be fungi, for example of the genera *Aspergillus* (for example the species *A. fumigatus, A. niger, A. ftavus, A. nidulans), Basidiobolus* (for example the species *B. microsporus, B. ranarum*), *Cephalosporium* (for example the species *C*. *chrysogenum, C. coremioides, C. diospyri, C. gregatum), Cryptococcus* (for example the species *C*. *neoformans, C. laurentii, C. albidus, C. gatrii), Histoplasma* such as *H. capsulatum, Pneumocystis* such as *P. jirovecii, Entomophthora, Skopulariopsis, Stachybotrys* such as *S*. *chartarum, Mucor, Rhizomucor, Absidia, Rhizopus, Altenaria, Stemphylium, Botrytis, Chrysosporium, Curvularia, Helmithosporium, Hemispora, Nigrospora, Paecilomyces, Phoma, Thielavia or Syncephalastrum,* or pathogenic yeasts of the genus *Candida,* for example the species. *C*. *albicans, C. guilliermondii, C. kruzei, C. parapsilosis, C. tropicalis.*

Microorganisms can also be protozoa, for example *Cryptosporidium parvum, Cryptosporidium hominis, Cryptosporidium serpentis, Entamoeba, Giardia, Toxoplasma gondii, Trypanosoma brucei, Trypanosoma cruzei, Plasmodium* species such as *P. falciparum, P. malariae*.

Accordingly, when the sample is taken from the host, advantageously a biological fluid, the nucleic acid molecules not contained into cells of the first subpopulation are free within this environment and nucleic acid molecules of microorganisms of the other subpopulation(s) representing a minor group, are packaged, especially encapsidated and/or enveloped or protected by the microorganism membrane(s).

In biological fluids (e.g. plasma, serum, urine), host nucleic acids are generally mainly found free within said biological fluids. As a consequence, depending on the starting sample, no step would be required to render host nucleic acid molecules free in order to later enable degradation of said nucleic acids molecules.

By "representing quantitatively a minor group" it is meant that the at least two subpopulations of nucleic acids present in the biological sample are unequally distributed within the assayed sample, the first subpopulation representing a large amount of molecules in view of the further subpopulation(s), which consist of smaller population(s) of molecules within the overall nucleic acids population of the assayed sample. The ratio between such a first and further subpopulations, especially the second subpopulation, can for example be of 1:10⁶. Such a ratio can be found in samples wherein the further subpopulation consists of nucleic acids of a pathogen whose detection within a sample mainly containing eukaryotic cellular DNA, especially genomic DNA, of the host is sought.

The ratio of 1:10⁶ referred to above is cited as example only. Depending on the abundance of the pathogen in a given sample, fluctuation of this ratio is likely to occur. This ratio compares total masses of the considered nucleic acid molecules populations in balance, taking into account both molecule sizes and abundance. In a particular embodiment, such a ratio can be based on a number of sequences reads.

The differential treatment applied to the first and further subpopulations consists in exclusively exposing the first subpopulation to conditions enabling a modification of the 3'- and/or 5'- extremities of its nucleic acid molecules, in particular DNA molecules in order to impair the ligation capacity and especially prevent the ligation of said nucleic acid molecules. Within the context of the invention, the further subpopulation nucleic acid molecules should mostly not be modified by this treatment, and accordingly retain ligation capacity in appropriate conditions.

According to a particular embodiment, the biological sample is exposed to degradation conditions to enable degradation of nucleic acid molecules that are not packaged *i.e.*, free nucleic acids and production of fragments thereof. In said degradation conditions, the packaged nucleic acid molecules of the microorganisms are not degraded.

By "free nucleic acid molecules" it is thus meant all the nucleic acid molecules that are accessible to the degradation conditions applied to the sample.

By "degradation conditions", it is meant any condition leading to breaking of nucleic acids. Such conditions can result from the use of an enzyme. Accordingly, "degradation conditions" involve enzymatic cleavage of nucleic acid molecules.

Use of enzymatic agents is however preferred: the biological sample can accordingly be treated with at least one nuclease, *e.g*. DNAse, resulting in the digestion of nucleic acid molecules and production of digested nucleic acid molecules thereof, in particular small digested nucleic acid molecules thereof.

The degrading conditions will preferably lead to the production degraded nucleic acid molecules, *i.e.* degraded nucleic acid molecules, or fragments especially digested nucleic acid molecules, with a length of about 100 nucleotides or more, but advantageously less than 2 kb. In a particular embodiment, degradation or digestion is not selective of specific nucleic acid sequences except that it should not degrade microorganism nucleic acids and thereof arise randomly within the nucleic acid molecules of the host providing the sample to be treated.

However, according to a particular embodiment, degradation conditions lead to nucleic acid fragments whose 3' and 5' extremities are not modified with respect to those of conventional nucleic acid molecules, *i.e.* their 3' and 5' extremities are competent to serve as a substrate for condensation resulting in a phosphodiester bond.

According to a particular embodiment, the free nucleic acid molecules mainly consist of the first subpopulation of nucleic acid molecules, as defined herein.

By contrast, within the context of the present invention, nucleic acid molecules that are not free are considered to be "packaged" nucleic acid molecules. According to a particular embodiment, wherein the microorganism is a virus, such packaged nucleic acid molecules are nucleic acids that are encapsidated within the viral capsid, which forms a protective barrier to such degradation conditions. Packaged nucleic acid molecules can also be protected by an envelope, such as a viral envelope especially adding to capsid. The envelopes typically contain cell membranes elements, such as phospholipids and proteins, and may include viral glycoproteins.

Packaged nucleic acid molecules can also be found when the microorganism is a bacterium a yeast or a fungus, because bacteria yeasts or fungi cell walls are generally more resistant and less penetrable for external agents than eukaryotic membranes. According to a particular embodiment, "packaged" nucleic acid molecules are therefore protected by a structure that is similar to a cell membrane, that separates the microorganism nucleic acids from the surrounding medium, said structure being resistant to disruption in the conditions of the invention applied to the nucleic acid of the host prior to or during modification of the 5' and 3' extremities, and in particular to digestion enzymes and therefore capable to contain the "packaged" nucleic acid molecules within a compartment, so as not to release the nucleic acids in the surrounding medium in the degradation conditions of the invention.

According to a particular embodiment, the "structure" separating the microorganism nucleic acids from the surrounding medium can be a membrane.

According to a particular embodiment, lysis conditions that are efficient for lysing non-microorganism cell walls or membranes are substantially not efficient for damaging the "package" structure so as to release its nucleic acids contents in the surrounding medium.

In a particular embodiment, the invention therefore relates to a method wherein the biological sample is obtained from a host infected with a microorganism, wherein the first nucleic acids subpopulation substantially consists of the higher eukaryotic host cell's genomic nucleic acid molecules, e.g. host's genomic DNA (gDNA), and the further nucleic acid subpopulation substantially consists of microorganism's nucleic acid molecules present in the biological sample as protected nucleic acid molecules, such as encapsidated microorganism nucleic acid molecules, or microorganism nucleic acid molecules contained in a virus particle or fungus or bacteria or yeast or parasite cell.

By "fragment" or "degraded acid nucleic molecules", it is meant nucleic acid molecules having preferably a length of 10 to 100, 200, 300, 500, 1000 and in general less than about 2000 nucleotides.

Modification of the 3'- and/or 5'- extremities of nucleic acid molecules in order to impair or advantageously prevent their ligation capacity can be achieved by several methods, one of which is the use of a phosphatase, for example a polynucleotide 5'-phosphatase to remove a phosphate group from the 5'-extremities of nucleic acids, especially DNA, which will remain with a 5'- free hydroxyl group.

"Modification at the 3'- extremity" or "modification at the 5'- extremity" means a modification occurring at the free end of the 3'- end, or 5'- extremity, respectively.

Modification at the 5'- extremity of polynucleotides can be achieved by using a polynucleotide 5'-phosphatase to remove a phosphate group from the 5'-extremities of nucleic acids, as described above.

Modification at the 3'- extremity of polynucleotides can also be achieved using conventional method providing nucleotides that lack a free 3'- hydroxyl group. One example is the addition at said 3'- extremity of polynucleotides of at least one nucleotide, in particular modified nucleotide, having an altered ligation capacity, as defined below. Such an addition can be performed through a polymerase, in particular a DNA polymerase (*e.g*. terminal nucleotidyl transferase, thermostable or thermolabile DNA polymerase), using notably the tailing DNA polymerase activity. Examples of modified nucleotides include ddNTP (2'-3'-dideoxynucleotide having a 3'-H extremity) or nucleotides coupled with chemical moieties impairing the ligation of the 3'- extremity of said nucleotides.

Modification of the 3'- and/or 5'- extremities of nucleic acid molecules can also be achieved through the ligation of adaptor(s) to said 3'- and/or 5'- extremities of polynucleotides. The extremities of said adaptors can be non-modified or modified extremities, said adaptors having however an altered ligation capacity, as defined below. A ligation step by a ligase, such as a DNA ligase, is required for performing such an embodiment. Adaptors can be synthetic oligonucleotide adaptors.

According to a particular embodiment of the invention, more than one method/technique of modification of the 3'- and/or 5'- extremities of nucleic acid molecules are used on a same sample when performing the invention, concomitantly or not.

Importantly, the modification carried out functionally impairs and advantageously prevent the capability of the modified nucleic acids fragments to form phosphodiester bonds. Modified nucleic acid molecules are therefore not able to link to other nucleic acid molecules of the sample, such as digested nucleic acid molecules, through their modified extremities.

By "ligation" capacity alteration or failure it is meant that the modified nucleic acids molecules are rendered incapable to serve as substrate for the formation of phosphodiester bonds linking 5'-extremity of one nucleic acid molecule to 3'-extremity of another nucleic acid molecule, in particular with other modified nucleic acids molecules, but also with non-modified nucleic acids molecules, present in the same sample or mixture.

In a particular embodiment, the step of modification of the 3'- and/or 5'-extremities of the nucleic acid molecules (step b.) is achieved through contacting the biological sample with a phosphatase enzyme in conditions enabling 5'-dephosphorylation of DNA.

According to a particular embodiment, the above-mentioned modifications result from any treatment conducted before microorganisms nucleic acids extraction from samples, thereby only modifying host-associated nucleic acids ends so as to prevent their ligation before amplification by MDA. In a specific embodiment, phosphatases can be used to remove 5'-Phosphate groups from DNA, RNA, ribo-and deoxyribonucleoside triphosphates.

Extraction of nucleic acid molecules as set forth in step c. of the above-mentioned method can be done through conventional techniques. This extraction step aims at recovering all the nucleic acid molecules of the sample and especially those of the further subpopulation and will generally require cell or capsid lysis for releasing the nucleic acids contained in the microorganism.

Mainly, two categories of methods of extraction of nucleic acid molecules from biological samples are known:
- The first one is based on lysis/extraction through organic solvents (e.g. Phenol/Chloroform extraction technique, Trizol...) followed by the precipitation of the extracted nucleic acid molecules in alcohol;
- The second one is based on lysis/extraction in the presence of strong concentrations of chaotropic agents/compounds/salts (*e.g*. Guanidium isothiocyanate, guanidine hydrochloride...), optionally assisted by the action of added detergents and/or enzymes (*e.g.* Proteinase K, lyzozymes...). Detergents might facilitate lysis but may not be indispensable. Extracted nucleic acid molecules are subsequently generally purified on affinity/resins columns, such as generally known in the art or sold in commercial kits.

These two categories of methods of extraction of nucleic acid molecules can be used jointly with physical treatments, which can be heating or sonication (non-exhaustive list), resulting in multisteps nucleic acid molecules extraction methods.

Chemical lysis can be achieved for example by using sodium dodecyl sulphate or guanidium thiocyanate, or hydroxide electro-generation-induced cell lysis, or alkaline buffers. Multisteps methods including heating might be required, or mechanical disruption methods, such as sonication. Another method is the use of proteinase K digestion in the absence or presence of detergents, phenolchloroform extraction followed by alcohol precipitation. DNA capture can also be achieved on silica-based resins or glass matrices or capture membranes.

Gram+ bacteria cell wall is however a membrane considered as being difficult to degrade. Therefore, when Gram+ bacteria presence is suspected, extraction/lysis of nucleic acid molecules requires hard extraction/lysis conditions involving for example mechanical disruption using beads and/or enzymatic digestion with endoglycosidases such as lysozyme.

For example when "packaged" nucleic acid molecules are contained within Gram+ bacteria, *i.e.* they are enveloped by the external Gram+ bacteria membrane, destruction of the packaging structure would involve using Zirconium/Silicium beads or other means such as lysozyme treatment capable of lysing Gram+ bacteria membrane(s).

According to a particular embodiment, extraction is performed through enzymes capable to digest chromatin and/or pathogens, e.g. proteinase K.

According to a particular embodiment, the conditions for the extraction of nucleic acid molecules substantially do not damage the genomes that are extracted, in particular the microorganism genomes that are susceptible to be contained within the sample.

Conversion of nucleic acid molecules resulting from step c. that are not DNA molecules into cDNA or DNA molecules is necessary to further enable their ligation. Conventional techniques using a DNA polymerase with reverse transcriptase activity can be used to this end.

According to a particular embodiment, the ligation step (step d). is performed through conventional techniques and enables the recovery of DNA that is capable of being amplified, in particular by polymerases such as a polymerase favouring nucleic acid molecules of a high length (*e.g*. over 2000 nucleotides) as an amplification substrate, *e.g*. high-fidelity DNA polymerase with high displacement strand activity such as the phi29 polymerase or the Bst DNA polymerase.

Known polymerases include for example (non-exhaustive list) : phi29 DNA Polymerase, Bst DNA Polymerase, *Bsu* DNA Polymerase, 9°Nm™ DNA Polymerase, VentR (exo-) DNA Polymerase, Deep VentR (exo-) DNA Polymerase, Klenow Fragment (3' -> 5' exo-) polymerase.

According to a particular embodiment, the ligation step allows for the recovery of nucleic acid molecules having more than 2000 nucleotides.

This embodiment is particularly advantageous when high-fidelity DNA polymerase with displacement strand activity, in particular high displacement strand activity, is to be used for further amplification. For instance, the phi29 polymerase requires DNA fragments for amplification, and is more efficient on linear DNA fragments that are larger than 2000 nucleotides.

By "displacement strand activity", it is meant that the DNA polymerase having this property have the ability to displace downstream DNA encountered during synthesis. The strand displacement triggered by a DNA polymerase having such a property generates newly synthesized single stranded DNA templates enabling more primers to anneal. Further primers annealing and strand displacements occurring on the newly synthesized templates results in a hyper-branched DNA network. As a result of using polymerases with displacement strand activity, in particular with high-displacement strand activity, high yield of products is therefore achieved.

Among DNA polymerases known to date, the phi29 or Bst DNA polymerases show high efficiency (or degree) in triggering displacement strand activity, and are therefore referred to as having high-displacement strand activity. Such high displacement strand activity is particularly appropriate for MDA amplification. However, any polymerase having a displacement strand activity can be advantageously used within the present invention.

The phi29 DNA polymerase is a monomeric protein displaying structural and functional similarity to the Klenow fragment of the E. coli Polymerase I enzyme. The phi29 DNA polymerase can be used under isothermal conditions, for example around 30°C, and presents a high processivity rate with a low error rate, due to its proofreading activity. The strand displacement activity of the phi29 DNA polymerase allows to generating large ramified nucleic acids fragments that are over 7 or even 10 kilobases long. This enzyme is commonly used in MDA-based protocols, without the need of specific primers (random primers such as hexamers can be used).

The method of the invention can appropriately be used to enrich a sample in nucleic acids representative of the minor group or minor groups of nucleic acids in the sample, and therefore be part of a sample enrichment method through a subsequent amplification step on the basis of the sample prepared by the method of the invention.

The invention therefore also relates to a method for enrichment of a sample in nucleic acids representative of the minor group of nucleic acids in the sample, comprising the steps of:
a. performing the method of differential treatment according to the present invention, as described herein;
b. submitting the differentially treated sample to amplification steps or cycles to selectively amplify nucleic acid molecules of the further subpopulation of nucleic acid molecules contained in the sample, said amplification steps or cycles being performed using a polymerase preferably targeting nucleic acid molecules having a length of 2000 nucleotides or more as substrate, thereby selectively enriching the sample resulting from step a. in nucleic acids of quantitatively the minor group.

According to a particular embodiment, the amplification is performed using the phi29 DNA polymerase, in particular by Multiple Displacement Amplification (MDA).

Multiple Displacement Amplification (MDA) is a conventional amplification technique using the phi29 DNA polymerase. Reference is made to Berthet N, et al. (2008), BMC Mol Biol. 9:77. for an overview of this technique.

By enriching a sample in nucleic acids representative of one or many minor group(s) of nucleic acids, it is meant that the proportion of said nucleic acids representative of the minor group over the overall nucleic acids population in the sample can be increased to up to 2, 3, 4 or 5 logs. The log value represents the increase in concentration of the minor nucleic acid molecules group. The proportion of the nucleic acids representative of the minor group over the overall nucleic acids population can be monitored by using host and pathogen cellular marker genes, for example in real-time quantitative PCR experiments performed both on the original sample and the nucleic acid molecules amplified after performing the method of the invention on the original sample.

A particular application of the method of the invention concerns the preferential amplification of microorganism's, in particular viral, nucleic acids by MDA by decreasing background amplification of host nucleic acids.

The invention also relates to a method for *in vitro* detection of nucleic acid molecules, originating from microorganisms comprising the steps of:
a. performing a method for enrichment of a sample in nucleic acids of the present invention, as described herein, and
b. performing a step of qualitative and/or quantitative detection of enriched nucleic acid molecules within the enriched biological sample, for example by PCR-based techniques, microarrays or sequencing, or high-throughput sequencing methods.

According to a particular embodiment, the method of the invention allows for downstream identification of pathogens, for example with microarrays, HTS or specific PCRs.

The method of the invention can be used for detecting the presence of microorganism(s), in particular pathogenic microorganism(s), such as papillomaviruses, polyomaviruses, herpesviridae, lentiviruses (HIV), flaviviridae (WNF, YFV, dengue virus), measles virus, rabies virus or bacteria of the genera *Bacillus, Bordetella, Campylobacter, Chlamydia, Clostridium, Enterococcus, Escherichia, Helicobacter, Legionella, Leptospira, Listeria, Mycobacteria, Salmonella*, *Shigella, Staphylococcus, Streptococcus,* or *Aspergillus* fungi or pathogenic yeasts of the genus *Candida,* or parasite of *Plasmodium* species or mixtures thereof, within a biological sample. According to a particular embodiment, the assayed biological sample is susceptible to contain more than one microorganism nucleic acids. According to a particular embodiment, the assayed biological sample is susceptible to contain a very low concentration of microorganism(s) and includes a sample of non-biological material susceptible of being contaminated by abundant exogenous DNA before or after sampling. By "low" concentration, it is meant a concentration as low as 1/10⁸ or 1/10⁷, in particular 1/10⁶.

In a specific particular embodiment, the invention relates to an *in vitro* enrichment method for the preferential amplification of the quantitatively minor group of nucleic acid molecules subpopulation of a microorganism(s) over the genomic nucleic acid molecules subpopulation of a host, wherein both subpopulations are contained in a biological sample, comprising the steps of:
a. exposing the biological sample to degradation conditions by nucleases, to enable digestion of free nucleic acid molecules and production of digested nucleic acid molecules thereof;
b. exposing the biological sample to a phosphatase catalyzing the removal of 5'-Phosphate free groups of the digested nucleic acid molecules, in order to impair their ligation capacity;
c. extracting nucleic acid molecules of the sample, optionally after a step of elimination of the pre-treatment reagents used in steps a. and b., and thereby recovering digested nucleic acid molecule of the genomic nucleic acid molecules subpopulation having modified dephosphorylated 5'-extremities and the nucleic acid molecules of the microorganisms subpopulation having non-modified 5'- extremities;
d. optionally, if some nucleic acid molecules resulting from step c. comprise RNA molecule(s), performing a reverse transcription of said molecules into cDNA or DNA molecules,
e. performing a ligation step on nucleic acid molecules recovered from step c. and/or step d., in particular to obtain nucleic acid molecules having more than 2000 nucleotides in length,
f. submitting all nucleic acid molecules obtained from step e. to amplification by Multiple Displacement Amplification (MDA) using a high-fidelity DNA polymerase with displacement strand activity, in particular with high-displacement strand activity, such as the DNA polymerase of phage phi29, in conditions enabling amplification of nucleic acid molecules having a length of 2000 nucleotides or more.
g. optionally, detecting, in particular sequencing, for example by high-throughput sequencing (HTS), the amplified microorganism's nucleic acid subpopulation.

The invention also relates to a kit for performing a method of the invention, said kit comprising:
a. nuclease(s) and/or phosphatase(s), and,
b. optionally, means for performing nucleic acid extraction from a biological sample, and,
c. optionally, a high-fidelity enzyme with a strand displacement capacity, in particular a high strand displacement capacity, such as the phi29 DNA polymerase or the Bst DNA polymerase, and,
d. optionally, buffer(s) or reagent(s).

According to a particular embodiment, the high-fidelity enzyme used within the invention has a high strand displacement capacity, the level of which matches the level of activity, or is in the range covered by, or approaches the range covered by the disclosed strand displacement activity of the phi29 or the Bst DNA polymerases, which are considered as having high strand displacement capacities.

A kit of the invention can therefore comprise any one of the following elements:
- enzymes, and optionally reaction buffer(s) necessary to modify the 3'-and/or 5'- extremities of host nucleic acid molecules (*e.g*. nucleases and/or phosphatases and/or polymerases, with or without their reaction buffer(s), and/or modified nucleotides to be incorporated, if relevant. Details about elements which can be incorporated are given in the Examples section);
- nucleic acid molecules extraction kit(s);
- enzymes, and optionally buffer(s) and/or reagent(s) necessary for performing reverse transcription step(s) (e.g. reverse transcriptase, with or without buffer(s) and/or substrate nucleotides), or necessary for performing ligation step(s) (e.g. ligase enzyme, with or without buffer(s)), or necessary for performing amplification, in particular MDA amplification (e.g. enzyme having a displacement strand activity, as disclosed herein, with or without appropriate buffer(s), and/or appropriate primers and substrate nucleotide(s)).

Reference is made to present description, in particular to the Examples section below, for particular examples of elements that can be part of a kit according to the invention, taken alone or in all combinations thereof.

Such a kit may comprise instructions for performing any one of the methods of the invention, as described herein.

### EXAMPLE

The present invention results from the reasoning that selective impairment of the amplification of residual amounts of host DNA would favor the amplification of nucleic acids from microorganisms, in particular after a step of ligation of cDNA and DNAs performed to obtain longer nucleic acids on which a MDA will be performed.

**Table 3: Amplification of contaminating DNA (cellular beta-globin gene) is critically relying on the ligation step if nucleic acids have been pretreated with nucleases (short fragments): C_{T} of 26.66/26.51 and >45 with and without ligation, respectively. Amplification of untreated nucleic acids (long fragments > 2kb) does not rely on the ligation step (C_{T} of 24.97/25.00 and 26.94/26.99 with and without ligation, respectively).**

| | After extraction | | After amplification | | | |
|---|---|---|---|---|---|---|
| | | | With ligation | | Without ligation | |
| | Untreated | Treated nucleases + ends modification | Untreated | Treated nucleases + ends modification | Untreated | Treated nucleases + ends modification |
| Beta-Globin | 28.29/29.28 | >45/37.98 | 24.97/25.00 | 26.66/26.51 | 26.94/26.99 | >45/>45 |

As illustrated in Table 3, phi29 polymerase is less efficient on short nucleic acids, like those resulting from nucleases digestion, one way to achieve the goal of selective impairment of the amplification of residual amounts of host DNA is to introduce 5' or 3' -ends modifications to host DNA so as to impair the DNA ligation step. Because DNA ligase catalyzes the formation of the phosphodiester bond between the 5'-phosphate group (5'-P) and the 3'-hydroxyl group (3'-OH), 5'-dephosphorylated DNA, or DNA that do not contains a 3'-hydroxyl group will not ligate.

Because microorganisms nucleic acids are protected within viral particles or bacterial/fungal cells, treatments aiming at modifying DNA ends can advantageously be applied before the extraction of nucleic acids from microorganisms, allowing for selective modification of non microbiological nucleic acids. Thus a privileged sequence of steps is: 1) treatment of the sample with nucleases to digest host free nucleic acids 2) modification of 5' and/or 3' ends of remaining and digested host free DNA to impair further ligation 3) extraction of nucleic acids from the sample 4) Reverse transcription of pathogens RNA nucleic acids into DNAs Then ligation of nucleic acids 5) amplification by MDA 6) detection of microorganisms nucleic acids. Another aspect of the invention is to provide a method of sample preparation that minimizes the amount of steps necessary to obtain a sample enriched in pathogens nucleic acids, *e.g*. an "all-in-one" method, for example performed as a single step after pre-treatment and extraction of the contents of nucleic acids of the sample. In a particular embodiment pre-treatment and extraction of the contents of nucleic acids is also performed on a sample as single step, *i.e.* without additional, endogenous, means for isolating a particular subpopulation of nucleic acids of the sample, *e.g*. without involving further endogenous means, in particular no other endogenous means apart from the packaging structure present in the sample, for keeping microorganisms nucleic acids isolated from the host nucleic acids. In another aspect, such a "all-in-one" method might allow reverse-transcription of RNAs, if needed, ligation of host nucleic acids and amplification of the nucleic acids contents of the sample without additional manipulations of the sample in-between these steps, *i.e.* no further extraction step is required in-between these steps, which can therefore be performed consecutively on a sample. Dosage of nucleic acids can be performed on the treated sample at any time, in particular after pre-treatment and extraction and after an "all-in-one" ligation-amplification step.

According to a particular embodiment, a nucleic acids detection step according to the invention encompasses or is preceded by one or several nucleic acids extraction steps, as described herein.

As a proof-of-concept example, the inventors have used phosphatases that catalyze the removal of 5'-P groups from DNA, RNA, ribo- and deoxyribonucleoside triphosphates. Since treated fragments lack the 5' phosphoryl termini required by ligases, they cannot ligate through this extremity. The inventors have spiked a sample of plasma with Hepatitis E virus (HEV). This sample has been processed as follows:

### Treatment A:

1) treatment of the sample with nucleases to digest host free NAs
2) extraction of NAs from the sample
3) reverse transcription step
4) ligation of DNAs
5) amplification by MDA
6) quantitative real-time PCR detection of HEV cDNA and human DNA (β-globin gene)

### Treatment B: same as Treatment A with insertion of a dephosphorylation step between steps 1 and 2:

1) treatment of the sample with nucleases to digest host free NAs
2) treatment with a phosphatase
3) extraction of NAs from the sample
4) reverse transcription step
5) ligation of DNAs
6) amplification by MDA
7) quantitative real-time PCR detection of HEV cDNA and human DNA (β-globin gene)

An example of a standard operating procedure for performing such a "treatment B" is disclosed herein, leading to microorganisms nucleic acids isolation from acellular biological fluids followed by isothermal amplification (Phi29).

The following standard operating procedure describes the extraction of nucleic acids (NAs) of microorganisms (virus, bacteria, fungi, protozoas) from acellular homogeneous biological samples. It is sizable from few samples (performed in tubes) up to 96-well plates. Kits were used following the manufacturer recommendations.

### I. Material and reagents

- Nucleospin Dx Virus kit from Macherey-Nagel (Cat. No.: 740895.50) including Proteinase K (30mg)
- Baseline-ZERO DNAse from Epicentre/Illumina (Cat. No.: DB0711 K)
- Benzonase nuclease from Novagen (Cat. No.: 70664-3)
- 0.1 mm diameter Zirconia/Silica beads from Biospec (Cat. No.: 11079101 z)
- Absolute ethanol
- Antarctic Phosphatase from NEB (Cat. No.: M0289)
- SuperScript III First-Strand synthesis system for RT-PCR kit from Invitrogen (Cat. No.: 18080-051)
- QuantiTect Whole Transcriptome kit from Qiagen (Cat. No.: 207043)
- Quant-iT dsDNA BR assay kit from Invitrogen (Cat. No.: Q32853)
- 200µL microtubes
- RNAse- and DNAse-free 1.5ml Eppendorf tubes
- 20µL, 200µL and 100µL pipetman
- Tabletop centrifuge
- Thermocycler
- FastPrep FP 120 from Qbiogene
- Nanodrop 8000 from Thermo scientific
- Qubit fluorometer from Invitrogen

### II. Extraction of nucleic acids

This procedure uses the Nucleospin Dx Virus kit from Macherey-Nagel.

Data provided in Tables disclosed within the present application were obtained using the specific protocol disclosed here-below.

### A. Reagent preparation

- The lyophilized Proteinase K (30mg) is resuspended in 1.35ml of PB buffer (provided with the enzyme)
- When using the Nucleospin Dx Virus kit for the first time, dissolve the carrier RNA into 1ml of RAV1 buffer, and transfer back the obtained solution to the RAV1 bottle solution
- Add 50ml of absolute ethanol to the RAV3 buffer solution

### B. Operating procedure

1. Preheat RE buffer solution (nucleic acid elution buffer used at the end of the extraction procedure) to 70°C in a sterile tube. 60µL of RE solution will be used per extraction
2. For each sample, mix in an Eppendorf tube 150µL of biological fluid sample (plasma for instance) with 17µL of Baseline-ZERO buffer (this buffer is optimal for the activity of the Baseline-ZERO enzyme. Baseline-ZERO buffer contains 10X concentration of 100 mM Tris HCl (pH 7.5), 25 mM MgCl₂ and 5 mM CaCl₂), 4µL of Benzonase enzyme and 4µL of Baseline-ZERO enzyme. Incubate for 2 hours at 37°C
3. Add 20µL of 10X Antartic Phosphatase buffer, mix and add 2µL of the Antartic Phosphatase enzyme. Incubate for 1 hour at 37°C
4. Add 600µL of RAV1 buffer (lysis buffer, lysing envelopes, membranes, capsids protecting pathogens nucleic acid molecules) containing the carrier RNA
5. Add 0.8gr of 0.1mm diameter Zirconia/Silica beads and shake twice 25 seconds at 6.5m/sec. with the FastPrep FP 120 instrument from Qbiogene. Put the sample on ice between each shaking procedure
6. Add 20µL of proteinase K (degrading proteins, in particular of envelopes, membranes, capsids of pathogens) and mix with pipetting up and down and vortexing for few seconds
7. Incubate 1min. minimum at room temperature (action of Proteinase K)
8. Incubate 5min. at 70°C and quickly spin down the tube
9. Add 600µL of absolute ethanol, vortex 10 to 15sec. and spin down the tube
10.Transfer 700µL onto a Nucleospin Dx Virus column set on a collecting tube
11. Centrifuge 1 min. at 8,000g minimum
12. Discard the flow through, and set the column on a new collecting tube
13. Repeat steps 9 to 11 with the sample remaining volume
14. Wash the column by adding 500µL of RAW buffer solution (eluting contaminants non-specifically bonded to the column)
15. Centrifuge 1 min. at 8,000g minimum
16. Discard the flow through, and set the column on a new collecting tube
17. Wash the column by adding 600µL of RAV3 buffer solution
18. Centrifuge 1 min. at 8,000g minimum
19. Discard the flow through, and set the column on a new collecting tube
20. Wash the column by adding 200µL of RAV3 buffer solution (eluting contaminants non-specifically bonded to the column)
21.Centrifuge 3 min. at 11,000g
22. Discard the flow through, and set the column on a 1.5ml RNAse and DNAse-free tube
23.Add 50µL of RE buffer solution preheated to 70°C onto the column. Incubate 1-2min. on the bench
24. Centrifuge 1min. at 11,000g
25. Discard the column and close the tube containing the nucleic acids. The solution can be kept on ice or at 4°C for immediate use. Otherwise, it should be stored at -20°C (up to one month) or -80°C (longer periods)

### III. Quantification of nucleic acids

1. Concentration of nucleic acids is measured using 1µL of the sample collected at step II.B.25.
2. Measurements are performed on a Nanodrop 8000 equipment at 260 and 280nm. The ratio OD₂₆₀/OD₂₈₀ must be within the 1.6 to 1.8 range

### IV. Whole Transcriptome Amplification (WTA)

### A. Reverse transcription of extracted RNAs

The first-strand cDNA synthesis is performed with the SuperScript III First-Strand synthesis system for RT-PCR kit from Life Technologies. No more than 1µg of nucleic acids (from step II.B.25) in a maximum of 9µL volume are used.
1. For each sample, in a 200µL RNAse- and DNAse-free microtube add up to 1µg of nucleic acids collected at step II.B.25 and complete to 4µL with DEPC-treated water
2. Add 1µL of a mix containing 0.5µL of 10mM of each dNTPs and 0.5µL of 50mM random hexamers. Mix well
3. Heat sample at 75°C for 5min. and quickly place on ice for 2min.
4. Add 5µL of a mix containing:
   - 1µL of 10X RT buffer
   - 2µL of 25mM MgCl₂
   - 1µL of 10mM DTT
   - 0.5µL of RNAseOUT at 40U/ml
   - 0.5µL of SuperScript III RTase
5. Place each microtube in a thermocycler and incubate 10min. at 25°C (annealing and elongation of primers), 90min. at 45°C (first-strand cDNA synthesis) and 5min. at 95°C (enzyme inactivation)
6. The cDNAs obtained are used immediately or stored at -20°C for later utilization

### B. Ligation and amplification of cDNAs

The Multiple Displacement Amplification (MDA) reaction using the bacteriophage Phi29 DNA polymerase is only efficient on long linear (> 2kb) and circular molecules. Therefore it is necessary to ligate the short linear cDNAs obtained at step IV.A.6 into concatemers to obtain good amplification yields by the Phi29 enzyme. The Quantitect Whole Transcriptome kit from Qiagen is used for this procedure.
- Carefully vortex all buffers before use
- Ligation and amplification mixes must be prepared extemporaneously
- Be careful when pipetting the "ligation reagent" due to its extreme viscosity
   1. To 10µL of each cDNA sample obtained at step IV.A.6 add 10µL of a mix containing:
      - 6µL of ligation buffer
      - 2µL of ligation reagent
      - 1µL of ligation enzyme 1
      - 1µL of ligation enzyme 2
   2. Mix well and incubate 2hours at 22°C
   3. Add 30µL of a mix containing:
      - 29µL of REPLI-g Midi reaction buffer
      - 1µL of REPLI-g Midi DNA polymerase
   4. Mix well and incubate 8hours at 30°C
   5. The reaction is stopped by incubating tubes 5min. at 95°C

The amplified DNA is kept at +4°C if used within a month or at -20°C for longer periods.

### V. Quantification of amplified DNAs

Dosage of amplified DNAs is performed using the Quant-iT dsDNA BR assay kit from Invitrogen.
1. For each sample prepare 200 µL of Quant-iT dsDNA BR working solution by mixing 1µL of Quant-iT dsDNA BR reagent and 200µL of Quant-iT dsDNA BR buffer
2. To 10µL of each amplified DNA sample diluted ten times (1/10) add 190µL of Quant-iT dsDNA BR working solution
3. To 10µL of each λ DNA standard provided with the kit add 190µL of Quant-iT dsDNA BR working solution. The standard curve obtained from these measurements is used to determine the concentration of amplified DNA in the test samples

The standard operating procedure of the invention encompasses using means having similar purposes and functions as those described above and herein for performing a "treatment B". The operating procedure of the invention therefore also encompasses slight variations in the agents used, and may be differently summarized by the succession of the following steps:

| **Sample (*e*.*g*. plasma)** | **Reagent (Supplier)** | **Treatment** | **Conditions** |
|---|---|---|---|
| | Nuclease (various) | Digestion of free nucleic acids | 37°C |
| | Phosphatase (NEB) Terminal Transferase (NEB) | 5'-end dephosphorylation for phosphatase | 37°C for Phosphatase and Terminal Transferase |
| | *Taq* (various) | Template-independent polymerase activity for Terminal Transferase and Taq (3'-end tailing activity with a ddNTP) | Up to 72°C for Taq |
| | Zirconium/Silice beads (Biospec) | Mechanical lysis Gram⁺ | 2x |
| Extraction kit Qiagen or Macherey-Nagel | Proteinase K (Qiagen or Macherey-Nagel) | Digestion of chromatin and pathogens | Few min. Room temperature 5 min. 70°C |
| Extraction kit Qiagen or Macherey-Nagel | Cador pathogen kit (Qiagen) Nucleospin Dx Virus (Macherey-Nagel) | Purification of nucleic acids | 4x Centrif. 1 min >= 8000 g 1x Centrif. 3 min >= 11000 g |
| Extraction kit Qiagen or Macherey-Nagel | Cador pathogen kit (Qiagen) Nucleospin Dx Virus (Macherey-Nagel) | Elution of nucleic acids | 1-2 min. Room temperature (buffer heated at 70°C) 1x Centrif. 3 min >= 11000 g |
| | Nanodrop 8000 (Thermo scientific) | Dosage of nucleic acids | Room temperature |
| Reverse transcription kit (Life Technologies) | SuperScript III first-strand synthesis system for RT-PCR kit (Life Technologies) | Reverse transcription of RNAs from pathogens | 5 min. 75°C, 2 min. +0°C 10 min. 25°C, 90 min. 45°C 5 min. 95°C, 5 min. +0°C |
| QuantiTect Whole Transcriptome kit (Qiagen) | Mix of ligases (Qiagen) | Concatemers of single strands nucleic acids | 2 hours 22°C |
| QuantiTect Whole Transcriptome kit (Qiagen) | Phi29 (Qiagen) | Amplification of DNA (circular and linear longer than 2000 bases) | 8 hours 30°C 5 min. 95°C |
| Quant-iT dsDNA BR assay kit (Life technologies) | Qubit fluorometer (Life Technologies) | Dosage of amplified nucleic acids | Room temperature |

### RESULTS

As shown in **Table 4,** the relative increase of viral versus cellular NA is 207 times, when the treatment A is used as a reference. As expected, the phosphatase step does not impact significantly this ratio in the absence of the MDA step. This shows that the inhibition of ligation of short nucleic acids, as cellular DNA following the nuclease treatment, should be the mechanism of action, as anticipated in the design of the experiment.

It can also be seen that the C_{T} of HEV was 40 (barely detectable), after direct extraction of the plasma sample. Direct amplification of the plasma sample led to a disappearance of the HEV signal, linked to the relative higher amplification of cellular DNA. Use of the combination of nuclease + phosphatase before amplification led to a mean C_{T} of 29.31 *i.e.* an amplification of HEV sequences of 6220 fold.

**Table 4:** Plasma spiked with HEV: Additive effects of modifications of DNA ends in the context of nuclease treated samples. The number of genome copies of cellular DNA was evaluated by quantitative real-time PCR (qPCR) targeted to the β-globin gene. The number of genome copies of viral RNA was evaluated by qRT-PCR (column "after extraction") or qPCR (column "after amplification") using primers targeted to the HEV genome. The experiments were repeated twice and PCRs were conducted one or two times. So 3 to 4 C_{T}S are depicted in each cell.

Conditions were as follow: Untreated sample: "untreated"; Treatment A: "treated nuclease"; Treatment B: "treated nuclease + ends modification". As expected, the phosphatase treatment (treatment B) does not change the C_{T} values before the MDA procedure (compare columns "after extraction" in conditions "treated nuclease" and "treated nuclease + ends modification") for both genes, but the mean C_{T} for the viral genome is significantly decreased (from 35.62 to 29.31) following MDA amplification (compare columns "after amplification" for conditions "treated nuclease" and "treated nuclease + ends modification"). This means that viral sequences were preferentially and selectively amplified over cellular sequences, showing that end-modifications of cellular NAs brought by phosphatase prevented ligation and subsequent amplification.

Other results are provided in Tables 5 to 7, resulting from experiments aimed at evaluating the concentration of target (pathogens nucleic acids) relative to gDNA (host cell nucleic acids) after amplification (qPCR).

**Table 5:** Partial hydrolysis of host free DNA with nucleases and differential amplification of cellular versus viral nucleic acids. A plasma sample was spiked with HEV. As in Table 4, the number of genome copies of cellular DNA was evaluated by quantitative real-time PCR (qPCR) targeted to the β-globin gene. The number of genome copies of viral RNA was evaluated by qRT-PCR (column "after extraction") or qPCR (column "after amplification") using primers targeted to the HEV genome. The experiments were repeated twice and PCRs were conducted one or two times. Only mean C_{T}S are depicted in each cell.

The differences between cellular and viral C_{T}S are depicted. Negative figures correspond to a higher concentration of cellular DNA than HEV DNA or cDNA for the same PCR efficiency. The increase in relative concentration of viral versus cellular nucleic acids is depicted. The same conclusions drawn for Table 4 apply to Table 5. Results provided in Table 4 and Table 5 come from two identical but independent experiments. Said results demonstrate that modification of the cellular nucleic acid extremities prevent their further ligation/amplification and therfore favour virus sequences amplification.

**Table 6:** Whole treatment: plasma spiked with HEV (single-stranded RNA virus) and CAV (Canine adenovirus, double-stranded DNA virus). Legends are similar to those described for Table 4 with the modification that CAV data are provided here. Conclusions already drawn for Table 4 and 5 data also apply here. The introduction of the nucleases + phosphatase treatment ("treated nucleases + ends modification") in the sample preparation procedure led to a selective amplification of HEV and CAV viruses over cellular nucleic acid sequences. Albeit HEV and CAV pathogens were barely and no detectable after amplification in the untreated sample (mean C_{T}S of 35.36 and >45, respectively), they were readily detectable in the treated sample (mean C_{T}S of 37.8 and 25.9, respectively). In the meantime, the cellular β-globin gene readily detected in the untreated sample (mean C_{T} of 32) was undetected in the treated sample (mean C_{T}>45). This selective amplification of pathogens target sequences is shown by the relative amplification values (target/cellular amplification) on the far right column.

Table 7A and 7B: Whole treatment: Plasma spiked with HEV (single-stranded RNA virus) and FPV (Feline parvovirus, single-stranded DNA virus) - Two independent experiments are shown. With the modification of the FPV, the legends and conclusions of Table 6 apply to the current Table. Table 7 experiments are independent from Table 6 experiment.

**Table 4**

| Ratio target/cell DNA after extraction (log) | | Untreated | | Treated nuclease | | Ratio target /cell DNA after amplification (log) relative to untreated | Treated nuclease + ends modification | | Radio target/cell DNA after amplification (log) |
|---|---|---|---|---|---|---|---|---|---|
| | | | | | | | | | **amplification relative to untreated** |
| | | After extraction | After amplification | After extraction | After amplification | | After extraction | After amplification | |
| | Beta-globlin | 26.05/26 03 | 25 14/25.09 | 35.86/35 80 | 34.59/34.61 | | 35.75/36 10 | 34,17/34 27 | |
| | | | | 35.59/35.46 | 30.31/30 54 | | 36.25/35,23 | 33.80/33.69 | |
| -47 | VHE | 40.00/40.00 | >45/>45 | 36.75/36.67 | 36.33/38.49 | -1.03 | 37 24/36.30 | 29.64/29 50 | 1.6 |
| | | | | 37 14/36.64 | 33.66/33 99 | 3.7 | 34.38/34.49 | 28.80 | **6,3** |

**Table 5**

| | | Treated nucleases | | Treated nuclease + ends modifications | | |
|---|---|---|---|---|---|---|
| Ratio target/cell DNA after extraction (log) | | After extraction | After amplification | After extraction | After amplification | Ratio target/cell DNA after amplification (log) |
| | | | | | | **amplification relative to untreated** |
| | Beta-Globin | 34,3 | 27,3 | 33,8 | 26,3 | |
| | | | | | | 2,8 |
| | Hepatitis E virus | 36,7 | 30,7 | 36,0 | 23,5 | **3,6** |
| | | | | | | |

**Table 6**

| | | Untreated | | Treated nucleases + ends Modification | | |
|---|---|---|---|---|---|---|
| Ratio target/cell DNA after extraction (log) | | After extraction | After amplification | After extraction | After amplification | Ratio target/cell DNA after amplification (log) |
| | | | | | | **Relative amplification** |
| | Beta-Globin | 35,99/36,20 | 32,83 / 31,15 | >45 / >45 | >45 / >45 | |
| | | | | | | >2 |
| <-3 | Hepatitis E virus | >45/>45 | 35,37 / 35,34 | 37,92 / 38,01 | 37,87 / 37 74 | **>5** |
| <-3 | | | | | | |
| | Canine adenovirus | >45 / >45 | >45 / >45 | 25,95 / 26,52 | 25,86 / 25,95 | >6.3 |
| | | | | | | **>9,3** |

**Table 7A**

| | | Untreated | | Treated nucleases + ends modification | | | |
|---|---|---|---|---|---|---|---|
| Ratio target/cell DNA after extraction (log) | | After extraction | After amplification | extraction | After amplification | Rato target/cell DNA after amplification (log) | |
| | | | | | | **Relative amplification** | |
| | | | | | | | |
| | Beta-Globin | 31,9/32,1 | 35 1/34,4 | 40,0*/38,6 | >45/>45 | | |
| | | 32,6/32,3 | 37,2/>45 | 37,3/38,1 | >45/>45 | | |
| -1.2 | | | | | | | |
| | HEV | 36 4/37 0 | 37,0/35.8 | 40,0*/40,0* | 35,5/35,3 | 32 | **4,8** |
| -1.0 | | 37.0/35,8 | 1 | >45/39,6 | 35,7/35,7 | 3,1 | **4,4** |
| -43 | | | | | | | |
| -4.5 | Parvovirus | >45/>45 | 27.6/28,0 | >45/>45 | 29.2/29,0 | 5.3 | **9.6** |
| | | >45/>45 | 27.3/27,5 | >45/>45 | 288/28,9 | 54 | **9,9** |

**Table 7B**

| | | | Untreated | | Treated nuclease + ends modifications | | |
|---|---|---|---|---|---|---|---|
| Ratio target/cell DNA extraction (log) | | | | | | | ratio target/cell DNA after amplification (log) **Relative amplification** |
| | | | After extraction | After amplification | After extraction | After amplification | |
| | | Exp#1 | 32.96/33, 14 | **36,44/36,79** | 40,00*/38, 14 | **>45>45** | |
| | B-Globine | Exp#2 | 33.46/33,36 | **31,73/>45** | 37.79/39,36 | **>45/>45** | |
| -1.2 | | Exp#1 | 37,40136,49 | **35,52/35,19** | 37,68/39,23 | **36,66/35,05** | 30 **4.2** |
| -10 | HEV | Exp #2 | 36, 13/36,61 | **35,20/35,75** | 36,01/35,66 | **35,48/35,59** | 32 **4.2** |
| -39 | | Exp #1 | >45/>45 | **28,51/28,46** | >45/>45 | **28,49/28,13** | 56 **9.5** |
| -3.8 | Parvovirus | Exp.#2 | >45/>45 | **27,99/27,83** | >45/>45 | **27,78/27,61** | 5.8 **9.6** |
| | | | | | | | |

## Claims

1. A method for *in vitro* differential treatment of nucleic acid molecules contained in a biological sample and comprising a first subpopulation of free nucleic acids of higher eukaryotic cells, such as genomic nucleic acids, in particular genomic DNA, and at least one further subpopulation of nucleic acids of microorganism(s) wherein said microorganism(s) nucleic acids are packaged nucleic acids that represents quantitatively a minor group of the nucleic acids of the sample, comprising the steps of:
a. optionally, exposing the biological sample to degradation conditions to enable degradation of free nucleic acid molecules and production of degraded nucleic acid molecules thereof;
b. exposing the biological sample to conditions enabling the modification of the 3'- and/or 5'- extremities of degraded nucleic acid molecules of the first nucleic acids subpopulation in order to impair their ligation capacity;
c. extracting nucleic acid molecules of the sample and thereby recovering degraded nucleic acid molecules of the first nucleic acids subpopulation having modified 3'- and/or 5'- extremities and nucleic acid molecules of the further subpopulation having non-modified 3'- and/or 5'- extremities;
d. optionally, converting nucleic acid molecules from step c. that are not DNA molecules, into cDNA or DNA molecules,
e. optionally, performing a ligation step on nucleic acid molecules recovered in step c. and/or step d. to obtain nucleic acid molecules having more than 2000 nucleotides in length.

2. The method of claim 1, wherein the step of modification of the 5' extremities of the degraded nucleic acid molecules in step b. is achieved through treating the biological sample with a phosphatase enzyme enabling 5'-dephosphorylation of DNA.

3. The method of any one of claims 1 or 2, wherein the biological sample comprises microorganisms such as viruses, bacteria, yeasts, fungi or parasites, especially pathogenic microorganisms.

4. The method of any one of claims 1 to 3, wherein the biological sample is obtained from a host infected with a microorganism, and wherein in said sample the first nucleic acids subpopulation substantially consists of genomic nucleic acid molecules of higher eukaryotic host cells, e.g. host genomic DNA (gDNA), and the further nucleic acid subpopulation substantially consists of packaged nucleic acid molecules of microorganisms, such as encapsidated and/or enveloped nucleic acid molecules, or nucleic acid molecules that are contained within viral particles or bacteria or yeast or fungal cells or parasites.

5. A method for enrichment of a sample in nucleic acids representative of the minor group of nucleic acids in the sample, comprising the steps of:
a. performing the method of differential treatment according to any one of claims 1 to 4 on a sample;
b. submitting the differentially treated sample to amplification steps or cycles to selectively amplify nucleic acid molecules of the further subpopulation of nucleic acid molecules contained in the sample, said amplification steps or cycles being performed using a polymerase preferably targeting nucleic acid molecules having a length of 2000 nucleotides or more as substrate, thereby selectively enriching the sample resulting from step a. in nucleic acids of quantitatively the minor group.

6. The method according to claim 5, wherein amplification is performed using a DNA polymerase with displacement strand activity, in particular by Multiple Displacement Amplification (MDA).

7. The method according to claim 6, wherein the DNA polymerase has a high- displacement strand activity, in particular is the phi29 polymerase or the Bst DNA polymerase.

8. A method for *in vitro* detection of nucleic acid molecules, originating from microorganisms comprising the steps of:
a. performing a method for enrichment of a sample in nucleic acids according to any one of claims 1 to 7, and
b. performing a step of qualitative and/or quantitative detection of enriched nucleic acid molecules within the enriched biological sample, for example by PCR-based techniques, microarrays or sequencing, or high-throughput sequencing methods.

9. The method of any one of claims 1 to 8, for detecting the presence of microorganism(s), in particular pathogenic microorganism(s), such as papillomaviruses, polyomaviruses, herpesviridae, lentiviruses (HIV), flaviviridae (in particular WNV, YFV, dengue virus), measles virus, rabies virus or bacteria of the genera *Bacillus, Bordetella, Campylobacter, Chlamydia, Clostridium, Enterococcus, Escherichia, Helicobacter, Legionella, Leptospira, Listeria, Mycobacteria, Salmonella, Shigella, Staphylococcus, Streptococcus, or Aspergillus* fungi or pathogenic yeasts of the genus *Candida,* or parasite of *Plasmodium* species or mixtures thereof, within a biological sample.

10. An *in vitro* enrichment method according to any one of claims 1 to 9, for the preferential amplification of the quantitatively minor group of a nucleic acid molecules subpopulation of microorganism(s) over the genomic nucleic acid molecules subpopulation of a host, wherein both subpopulations are contained in a biological sample, comprising the steps of:
a. exposing the biological sample to degradation conditions by nuclease(s), to enable digestion of free nucleic acid molecules and production of digested nucleic acid molecules thereof;
b. exposing the biological sample to a phosphatase catalyzing the removal of 5'-Phosphate groups of the digested nucleic acid molecules, in order to impair their ligation capacity;
c. extracting nucleic acid molecules of the sample and thereby recovering digested nucleic acid molecule of the genomic nucleic acid molecules subpopulation having modified dephosphorylated 5'- extremities and the nucleic acid molecules of the microorganisms subpopulation having non-modified 5'-extremities;
d. optionally, if nucleic acid molecules resulting from step c. comprise RNA molecule(s), performing a reverse transcription of said molecules into cDNA or DNA molecules,
e. performing a ligation step on nucleic acid molecules recovered from step c. and/or step d., in particular to obtain nucleic acid molecules having more than 2000 nucleotides in length,
f. submitting nucleic acid molecules obtained from step e. to amplification by Multiple Displacement Amplification (MDA) using the a DNA polymerase with strand displacement activity, in conditions enabling amplification of nucleic acid molecules having a length of 2000 nucleotides or more.
g. optionally, detecting, in particular sequencing, for example by high-throughput sequencing (HTS), the amplified nucleic acid subpopulation.

11. The method of claim 10, wherein the DNA polymerase has a high-strand displacement activity, in particular is phi29 polymerase or Bst DNA polymerase.

12. A kit for performing a method according to any one of claims 1 to 11, comprising:
a. nuclease(s) and/or phosphatase(s), and,
b. optionally, a high-fidelity polymerase with strand displacement activity such as the phi29 DNA polymerase or Bst DNA polymerase, and,
c. optionally, buffer(s) or reagent(s).
